Europäisches Patentamt

European Patent Office

Office européen des brevets

(11) Veröffentlichungsnummer: **0 218 896**
**B1**

## (12) EUROPÄISCHE PATENTSCHRIFT

(45) Veröffentlichungstag der Patentschrift:
**29.08.90**

(51) Int. Cl.⁵: **C12P 5/02**

(21) Anmeldenummer: **86112271.1**

(22) Anmeldetag: **04.09.86**

(54) Verfahren zur Vorbehandlung von organischen Abfällen aus Schlachtungen.

(30) Priorität: **16.10.85 CH 4452/85**

(43) Veröffentlichungstag der Anmeldung:
**22.04.87 Patentblatt 87/17**

(45) Bekanntmachung des Hinweises auf die Patenterteilung:
**29.08.90 Patentblatt 90/35**

(84) Benannte Vertragsstaaten:
**AT BE DE FR GB IT SE**

(56) Entgegenhaltungen:
**EP-A- 0 105 943**
**GB-A- 524 315**

**CHEMICAL ABSTRACTS, Band 97, Nr. 1, Juli 1982, Seite 469, Zusammenfassung Nr. 4702f, Columbus, Ohio, US**
**CHEMICAL ABSTRACTS, Band 78, Nr. 2, 15. Januar 1973, Seite 226, Zusammenfassung Nr. 7607x, Columbus, Ohio, US**
**CHEMICAL ABSTRACTS, Band 46, Nr. 1, 10. Januar 1960, Zusammenfassung Nr. 7247b, Columbus, Ohio, US; L. LAVROV: "The conditions of high-quality processing of (slaughtering) by-products by chemical methods"**

(73) Patentinhaber: **GEBRÜDER SULZER AKTIENGESELLSCHAFT, Zürcherstrasse 9, CH-8401 Winterthur(CH)**

(72) Erfinder: **Lorré, Rudolf, Stefan-Lockner-Weg 26, D-5047 Wesseling(DE)**
Erfinder: **Strauch, Dieter, Prof.Dr.-med.vet., Jahnstrasse 32, D-7302 Ostfildern(DE)**

(74) Vertreter: **Sparing Röhl Henseler Patentanwälte European Patent Attorneys, Rethelstrasse 123, D-4000 Düsseldorf 1(DE)**

## Beschreibung

Die Erfindung betrifft ein Verfahren zur Vorbehandlung von organischen Abfällen, die bei Schlachtungen anfallen, insbesondere von Pansen-, Magen- und Darminhalten, bei welchem Verfahren aufgrund einer pH-Wert-Messung durch Zugabe eines Behandlungsmittels, insbesondere von Kalk, ein alkalischer pH-Wert eingestellt wird, ehe der Abfall einer anaeroben Fermentation zugeführt wird.

Es ist bekannt, Abwasser mit Hilfe einer anaeroben Fermentation zu reinigen; um vor dem Reaktionsbehälter für die Fermentation eine schnelle Flockenbildung zu erreichen, und eine Trennung von Feststoffen und Flüssigkeit in einem Absetzbecken zu verbessern, wird bei einem bekannten Verfahren (US-PS 2,66l,332) das Abfall/Wasser-Gemisch durch Zugabe von Kalk oder einem anderen, den pH-Wert steigernden Mittel alkalisch gemacht, wobei pH-Werte bis 9,5 erreicht werden sollen. In der Praxis hat sich dieses Verfahren nicht durchsetzen können, da einerseits seine Betriebsweise zu aufwendig ist, weil die Ausbeuten an verwertbarem Biogas nur gering sind; es werden im Mittel beispielsweise 250 l Biogas pro kg OTM (OTM: organische Trockenmasse) erhalten. Andererseits ist das die Fermentation verlassende Ausfaulsubstrat seuchenhygienisch nicht einwandfrei, da bei diesem Verfahren Keime und Krankheitserreger nicht mit Sicherheit abgetötet werden.

Aufgabe der Erfindung ist es daher, das genannte Verfahren - insbesondere für eine biologische Schlachthofentsorgung mittels anaerober Fermentation - in Richtung auf eine erhöhte Ausbeute an verwertbarem Biogas zu verbessern, und das Ausfaulsubstrat seuchenhygienisch unbedenklich zu machen.

Erfindungsgemäss wird diese Aufgabe dadurch gelöst, dass die Abfälle einer Zerkleinerung unterworfen werden, dass dem Feststoff/Wasser-Gemisch soviel Kalk zugegeben wird, dass ein pH-Wert von mindestens l2 eingestellt wird, dass ferner das auf diesem pH-Wert gehaltene Gemisch für eine Verweilzeit von mindestens 3 Stunden unter Rühren und Umwälzen gespeichert wird, und dass das Gemisch schliesslich einem anaeroben Bioreaktor zugeführt wird; die Kalkzugabe wird dabei beispielsweise in Form einer, vorzugsweise frisch zubereiteten l0%-igen, wässrigen Weisskalkhydrat-Lösung durchgeführt.

Es hat sich gezeigt, dass die Biogaserzeugung durch die Vorbehandlung entscheidend gesteigert wird; weiterhin gewährleistet die starke Basizität des vorbehandelten Gemisches, dass alle Keime und Krankheitserreger abgetötet werden. Entsprechend der erhöhten Biogasausbeute ist der Abbaugrad der organischen Feststoffmasse ebenfalls gesteigert.

Als weitere Vorteile des neuen Vorbehandlungsverfahrens bzw. als dessen Folge ergeben sich:
- eine Schwimmdeckenbildung aus Schwimmschlamm wird im Bioreaktor vermieden;
- die mechanischen Eigenschaften des Feststoff-Substrates im Bioreaktor sind verbessert, was eine vollständige Durchmischung des Substrats im Reaktor bewirkt;
- der Schwefelanteil im Biogas ist vernachlässigbar gering;
- die Trennung von Fermentationssubstrat und Flüssigkeit ist erleichtert;
- der hohe Abbaugrad an organischer Masse im Reaktor und die leichte Absetzneigung des Faulsubstrates ergeben nur noch geringe Faulschlammengen, die zu Entsorgen bzw. an die Landwirtschaft abzugeben sind;
- durch die Vorbehandlung des Substrates wird der Düngewert des Faulschlammes verbessert;
- der erhöhte Abbaugrad bewirkt verstärkt eine Umwandlung von organisch gebundenem Stickstoff in Ammonium-Stickstoff; dieser ist durch die Bodenflora schneller in pflanzenverfügbare Verbindungen umzuwandeln. Dadurch werden die Umweltbelastung durch Stickstoff bzw. die Nitratbelastung im Trinkwasser verringert;
- es treten keine Verstopfungsprobleme auf;
- die Pufferkapazität des Faulsubstrates wird erhöht, so dass der Fermentationsprozess stabilisiert und ein "Kippen" der Biologie vermieden wird.

Da die Vorbehandlung mit Vorteil bei erhöhten Temperaturen von 25 - 55° C, insbesondere bei 33° C, durchgeführt wird, ist es zweckmässig, dem Gemisch aus zerkleinerten Feststoff-Abfällen und Abwasser frisch zubereitete Kalkmilch zuzugeben, um die Reaktionswärmen der exothermen Lösungs- und Hydrataktions-Reaktionen zur Temperaturerhöhung des Gemisches zu nutzen. Falls es erforderlich ist, die Fliessfähigkeit des Feststoff/Wasser-Gemisches zu erhöhen, ist es möglich, diesem Verdünnungswasser zuzumischen, wofür mit Vorteil ein Teil der flüssigen Phase aus dem Bioreaktor verwendet wird, da sich diese Phase bereits auf einer erhöhten Temperatur befindet.

Als besonders günstig hat es sich erwiesen, wenn ein pH-Wert von mindestens l2,5 eingehalten wird.

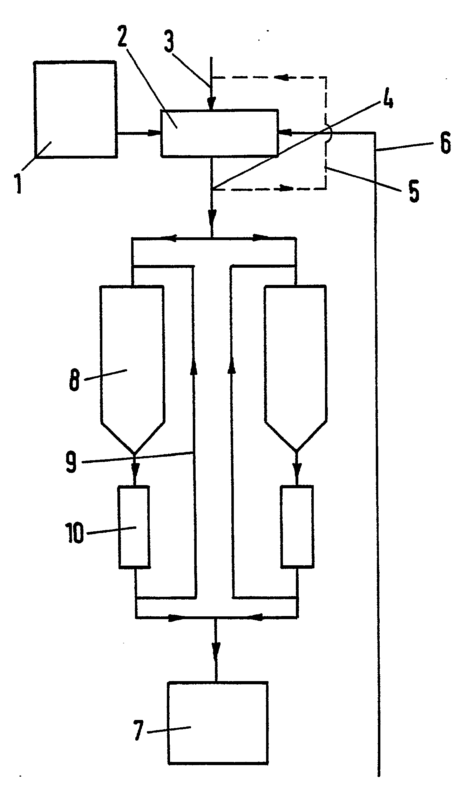

Im folgenden wird die Erfindung anhand eines Ausführungsbeispiels im Zusammenhang mit der Zeichnung näher erläutert.

Die einzige Figur zeigt schematisch ein Fliessschema für die Durchführung der erfindungsgemässen Vorbehandlung.

Schlachthofabfälle, die zuvor von anorganischen Fremdkörpern, wie z.B. Metallteilen, getrennt worden sind, werden in einem Sammelbehälter l gesammelt; ihr Hauptfeststoffanteil besteht aus Panseninhalten mit einem Trockensubstanzgehalt bis zu 20 %. Der Feststoffanteil anderer Darm- und Mageninhalte, beispielsweise aus Schweineschlachtungen, ist geringer, so dass durch Mischung aller Abfälle unter Umständen ein bereits ausreichend fliessfähiges Gemisch im Behälter l vorhanden ist.

Dieses wird einer bekannten Zerkleinerungsvorrichtung 2 zugeführt, die je nach Zusammensetzung des Feststoffanteils beispielsweise aus Matzeratoren, Mühlen und/oder Reiss-Schnecken bestehen kann. In ihr werden die Feststoffanteile des Gemisches soweit zerkleinert und zerrissen, dass die Teilchengrösse mindestens zum weitaus überwiegenden Teil, d.h. für 90 % des Feststoffinhaltes, unter l0 mm beträgt.

Zur Einstellung des vorzugsweise l2,5 betragenden pH-Wertes, der am Austritt der Vorrichtung 2, beispielsweise an der Stelle 4, gemessen wird, wird in die Vorrichtung 2 durch die Zuführung 3 frisch zubereitete Kalkmilch gegeben. Diese besteht bevorzugt aus einer l0%-igen Lösung von Weisskalk in Wasser. Es werden dabei beispielsweise pro kg OTM 60 - l50 g Kalziumhydroxid ($Ca(OH)_2$) zugegeben. Dessen Teilchengrösse beträgt mit Vorteil unter 0,4 µm. Eine derartig feine "Mahlung" in der Kalkhydrataufschlämmung hat sich als zweckmässig erwiesen, weil dadurch ein "Zusammenbacken" der Feststoffe zu grösseren Agglomeraten verhindert wird, die sich als Schlamm im nachfolgenden Bioreaktor 7 absetzen können und aus diesem Reaktor abgezogen werden müssen; die feinen Teilchen bleiben dagegen "in der Schwebe" und werden mit der Flüssigkeit aus dem Reaktor entfernt.

Die Temperatur in der Vorrichtung 2 - sowie in den nachfolgenden, noch zu beschreibenden Behandlungsstufen - wird auf 25 - 55°C, vorzugsweise auf 33°C, gehalten.

Ein pH-Wert-Messer an der Stelle 4 steuert dabei über eine Signalleitung 5 nicht dargestellte Dosierpumpe, durch die die Kalkmilch-Lösung in die Vorrichtung 2 eingespeist wird.

Ist die Fliessfähigkeit des Gemisches aus dem Behälter I ungenügend, so kann in die Vorrichtung 2 zusätzlich "Verdünnungswasser" über eine Einspeisung 6 zugegeben werden. Dieses Verdünnungswasser besteht aus der flüssigen Phase, die nach der anaeroben Fermentation im Bioreaktor 7 von dem Ausfaulsubstrat abgetrennt worden ist.

Das zerkleinerte, den geforderten pH-Wert aufweisende Gemisch gelangt aus der Vorrichtung 2 in einen Zwischenspeicher 8. In diesem wird es mit Hilfe nicht gezeigter Rührvorrichtungen gerührt und für mindestens 3 Stunden gespeichert; die Verweilzeit im Speicher 8 kann jedoch auch bis zu l2 Stunden betragen. Ueber eine Umwälzverbindung 9 kann der Speicherinhalt, beispielsweise durch nicht gezeigte Umwälzpumpen, zusätzlich umgewälzt werden.

Das nach frühestens 3 Stunden konditionierte und hygienisierte Feststoff/Wasser-Gemisch wird, falls erforderlich, in einer Vorrichtung l0 nochmals zerkleinert, ehe es zur Fermentation in den Bioreaktor 7 gelangt. Um für diesen einen kontinuierlichen Betrieb zu gewährleisten, sind der Speicher 8 sowie die Vorrichtungen l0 doppelt vorhanden und werden abwechselnd auf den Bioreaktor 7 geschaltet, der in bekannter Weise betrieben wird.

Die für die Förderung und Lenkung des Abfall/Wasser-Gemisches notwendigen Förder-, Absperr- und gegebenenfalls Steuer- und Regelorgane sind konventioneller Art und in dem Schema der Figur nicht dargestellt.

In einer bereits ausgeführten Versuchsanlage werden pro Tag etwa 50 kg eines Gemisches aus Pansen- und anderen Magen- und Darminhalten mit einem OTM-Gehalt bis zu 20 % der erfindgungsgemässen Vorbehandlung unterworfen. Durch die kalkmilchzugabe, und gegebenenfalls zusätzliches Verdünnungswasser, wird das anfallende Gemisch verdünnt; die Zerkleinerung der Feststoffanteile erfolgt bis zu Teilchen-von 0,5 bis l0 mm.

Die Wirksamkeit der neuen Vorbehandlung zeigt nachstehende Tebelle, bei der verschiedene Kenngrössen für die Beseitigung von Schlachthofabfällen durch eine anaerobe Fermentation ohne (Spalte I) und mit (Spalte 2) der neuen Vorbehandlung einander gegenübergestellt sind:

| Kenngrösse | Spalte 1 | Spalte 2 |
|---|---|---|
| Verfahren | Zerkleinerung der Feststoffe | Zerkleinerung der Feststoffe |
| | | Vorbehandlung |
| | Fermentation | Fermentation |
| Betriebsverhalten bei Fermentation | sehr labil | stabil |
| pH-Wert bei Fermentation | erheblich schwankend (5,6 - 7,6) | relativ konstant (7,4 - 7,7 |
| Verstopfungsgefahr | permanent | keine |
| Abbaugrad in % | 30 - 35 | 60 - 80 |
| Biogas-Ausbeute (l/kg OTM) | 180 - 260 | 400 - 650 |
| davon | | |
| $CO_2$-Anteil in Vol.% | 35 - 60 | 30 - 40 |
| Seuchenhygienisch unbedenklich | nein | ja |

## Patentansprüche

I. Verfahren zur Vorbehandlung von organischen Abfällen, die bei Schlachtungen anfallen, insbesondere von Pansen-, Magen- und Darminhalten, bei welchem Verfahren aufgrund einer pH-Wert-Messung durch Zugabe eines Behandlungsmittels, insbesondere von Kalk, ein alkalischer pH-Wert eingestellt wird, ehe der Abfall einer anaeroben Fermentation zugeführt wird, dadurch gekennzeichnet, dass die Abfälle einer Zerkleinerung unterworfen werden, dass dem Abfall/Wasser-Gemisch soviel Kalk zugegeben wird, dass ein pH-Wert von mindestens I2 eingestellt wird, dass ferner das auf diesem pH-Wert gehaltene Gemisch für eine Verweilzeit von mindestens 3 Stunden unter Rühren und Umwälzen gespeichert wird, und dass schliesslich das Gemisch einem anaeroben Bioreaktor zugeführt wird.

2. Verfahren nach Anspruch I, dadurch gekennzeichnet, dass ein Teil der flüssigen Phase aus dem Bioreaktor (7) zur Verdünnung des Abfall/Wasser-Gemisches in die Vorbehandlungsstufe zurückgeführt wird.

3. Verfahren nach Anspruch I, dadurch gekennzeichnet, dass die Kalkzugabe in Form einer wässrigen Weisskalkhydrat-Lösung durchgeführt wird.

4. Verfahren nach Anspruch I, dadurch gekennzeichnet, dass die Kalkzugabe in Form von gebranntem Kalk durchgeführt wird.

5. Verfahren nach Anspruch I, dadurch gekennzeichnet, dass ein pH-Wert von mindestens I2,5 eingestellt wird.

6. Verfahren nach Anspruch I, dadurch gekennzeichnet, dass während der Vorbehandlung eine Temperatur von 25 - 55° C, insbesondere von 33° C, eingehalten wird.

7. Verfahren nach Anspruch I, dadurch gekennzeichnet, dass das Gemisch vor dem Eintritt in den Bioreaktor (7) nochmals zerkleinert und homogenisiert wird.

## Claims

1. A method of pretreatment of organic slaughterhouse waste, more particularly of the contents of the paunch, stomach and intestine, in which method the pH is measured and made alkaline by adding an agent, more particularly lime, before the waste is sent for anaerobic fermentation, characterised in that the waste is comminuted sufficient lime is added to the mixture of waste and water to bring the pH to at least 12, the mixture is kept at this pH, agitated and circulated for a residence time of at least 3 hours, and finally the mixture is fed to an anaerobic bioreactor.

2. A method according to claim 1, characterised in that part of the liquid phase from the bioreactor (7) is returned to the pretreatment stage in order to dilute the mixture of waste and water.

3. A method according to claim 1, characterised in that lime is added in the form of an aqueous solution of hydrate of white lime.

4. A method according to claim 1, characterised in that the lime is added in the form of quicklime.

5. A method according to claim 1, characterised in that the pH is set at at least 12.5.

6. A method according to claim 1, characterised in that a temperature of 25–55°C, more particularly 33°C, is maintained during pretreatment.

7. A method according to claim 1, characterised in that the mixture is again comminuted and homogenized before entering the bioreactor (7).

**Revendications**

1. Procédé de prétraitement de déchets organiques qui sont produits lors d'abattages, notamment de contenus de panses, d'estomacs et d'intestins, procédé dans lequel on règle une valeur alcaline de pH par addition d'un agent de traitement, notamment de chaux, sur la base d'une mesure du pH avant que les déchets n'aient été conduits à une fermentation anaérobie, caractérisé en ce que les déchets sont soumis à une fragmentation, que de la chaux est ajoutée au mélange de déchets et d'eau en quantité suffisante pour ajuster un pH de valeur au moins égale à 12, que le mélange maintenu à cette valeur de pH est stocké pendant une période d'au moins 3 heures sous agitation et brassage, et que le mélange est enfin amené à un bioréacteur anaérobie.

2. Procédé suivant la revendication 1, caractérisé en ce qu'une partie de la phase liquide sortant du bioréacteur (7) est recyclée dans l'étape de prétraitement en vue de la dilution du mélange de déchets et d'eau.

3. Procédé suivant la revendication 1, caractérisé en ce que l'addition de chaux est effectuée sous forme d'une solution aqueuse d'hydroxyde de calcium.

4. Procédé suivant la revendication 1, caractérisé en ce que l'addition de chaux est effectuée sous forme de chaux calcinée.

5. Procédé suivant la revendication 1, caractérisé en ce qu'on ajuste une valeur de pH d'au moins 12,5.

6. Procédé suivant la revendication 1, caractérisé en ce qu'on maintient pendant le prétraitement une température de 25 à 55°C, notamment de 33°C.

7. Procédé suivant la revendication 1, caractérisé en ce que le mélange est encore divisé et homogénéisé avant son entrée dans le bioréacteur (7).